# EUROPÄISCHE PATENTSCHRIFT

(11) **EP 0 374 685 B1**
(45) Veröffentlichungstag und Bekanntmachung des Hinweises auf die Patenterteilung: **10.08.1994**
(21) Anmeldenummer: 89122879.3
(22) Anmeldetag: 12.12.1989
(51) Int. Cl.: C12Q 1/00, C12Q 1/28

(54) **Enzymatisches Nachweisgerät für Gase und Aerosole**
Enzymatic detection device for gases and aerosols
Dispositif de détection enzymatique pour les gaz et les aérosols

(30) Priorität: 17.12.1988 DE 3842607
(43) Veröffentlichungstag der Anmeldung: 27.06.1990
(73) Patentinhaber: Drägerwerk Aktiengesellschaft, 23542 Lübeck (DE)
(72) Erfinder: Rindt, Klaus-Peter, Dr., D-2400 Lübeck (DE); Scholtissek, Stephan, Dr., D-2400 Lübeck (DE)

(56) Entgegenhaltungen:
- EP-A- 0 142 690
- EP-A- 0 296 481
- WO-A-88/01299
- DE-A- 3 929 541
- JOURNAL OF CLINICAL CHEMISTRY AND CLINICAL BIOCHEMISTRY, Band 19, Nr. 7, 1981, US; B. POSTMANN et al., Seiten 435-439#

## Beschreibung

Die Erfindung betrifft ein Nachweisgerät für gasförmige Stoffe und Aerosole mit Hilfe einer enzymatischen Reaktion, mit den Merkmalen des Oberbegriffs des Patentanspruchs 1.

Bei einem enzymatischen Sensor für toxische Gase nach der EP-A2-142 690 ist ein Enzym (Acetylcholinesterase, AChE) kovalent an einem hydrophilen Träger gebunden. In einem Beutel ist das Enzymsubstrat aufgenommen, welches durch die Umgebungsluft mit dem Enzym als Katalysator hydrolysiert wird. Das Substrat wird über eine Filter-Membran-Kombination ständig zu dem Enzym-Träger geliefert, so daß die Hydrolyse ununterbrochen stattfinden kann, solange kein Cholinesterase-Hemmstoff in der zu untersuchenden Umgebung ist, welcher die Enzymreaktion hemmen würde. Die Hydrolyse wird dadurch zu einem elektrischen Meßsignal umgewandelt, daß an einer Meßelektrode das hydrolysierte Substrat oxidiert wird. Im bekannten Beispiel wird Acetylthiocholinperchlorat zu Salzsäure und Thiocholinperchlorat oxidiert. Der zwischen der Meßelektrode und einer Gegenelektrode gemessene Strom ist ein Maß für die Hydrolyse. Das Enzymsubstrat sowie der für die Oxidation erforderliche gepufferte Elektrolyt werden in einem Vorratsbehälter aufbewahrt, gelangen über einen Docht während der Bereitschafts- und Meßzeit ständig zu dem Substratbeutel und füllen dessen Verbrauch wieder auf.

Der bekannte Sensor prüft die Umgebungsluft auf die Anwesenheit von solchen Stoffen, welche die Enzymreaktion hemmen. Solche Enzyminhibitoren können unterschiedlicher Zusammensetzung sein (es gibt mehrere Cholinesterase-Inhibitoren in der angegebenen Klasse der phosphororganischen Verbindungen), so daß mit dem bekannten Sensor nur unspezifisch verschiedene Hemmstoffe nachgewiesen werden können. Der nachzuweisende Stoff ist nämlich nicht selbst das spezifische Enzymsubstrat, sondern dieses wird ständig durch den sensoreigenen lokalen Vorratsbehälter nachgeliefert.

In der älteren EP-A - 296 481 (zitiert unter Art. 54(3) + (4) EPÜ) wird ein Nachweisgerät beschrieben, welches in einem Gefäß ein Trägersubstrat besitzt, das das zum Nachweis erforderliche Enzym enthält. Vor einer Probenahme wird das Trägersubstrat mit einer Lösung getränkt, die alle für die nachfolgende Nachweisreaktion erforderlichen Bestandteile in der richtigen Zusammensetzung und Menge enthalten muß. Anschließend wird durch das getränkte Substrat die gasförmige Probe gesaugt, und je nach Menge des nachzuweisenden Stoffes erfolgt eine enzymatische Verfärbungsreaktion. Sollten jedoch die Bestandteile der Lösung, mit welcher das Trägersubstrat getränkt wird, in ihrer Menge nicht auf den nachzuweisenden Stoff abgestimmt sein, kann die Reaktion einerseits nicht vollständig ablaufen, wenn zu wenig Reaktionspartner in der Lösung sind, oder beim Durchsaugen der Probe wird teilweise Reaktionslösung mit entfernt, so daß andererseits die anfänglich ausreichende Menge an Lösung verarmt.

Des weiteren wird in der WO-A-8 801 299 ein Nachweisgerät vorgeschlagen, bei dem ein streifenförmiger Indikator-Träger in ein durchsichtiges Gefäß gesteckt wird, welches anschließend dem zu untersuchenden Stoff (z.B. Äthanoldampf) während einer vorbestimmten Zeit ausgesetzt wird. Der Indikator-Träger enthält alle die für eine enzymatische Reaktion erforderlichen Reaktionskomponenten. Diese müssen von vornherein in solchen Mengen vorliegen, daß der zu erwartende nachzuweisende Stoff vollständig mit ihnen abreagieren kann. Andernfalls findet eine unvollständige Verfärbungsreaktion statt, und das Meßergebnis kann nicht als repräsentativ für die nachzuweisende Probenmenge genommen werden.

Der vorliegenden Erfindung liegt somit die Aufgabe zugrunde, ein Nachweisgerät der genannten Art so zu verbessern, daß die Lösung an Reaktionskomponenten im Überschuß vorliegt und jeweils nur nach Bedarf zur Reaktion zwischen Enzym und dem als Enzym-Substrat anzusehenden nachzuweisenden gasförmigen Schadstoff gelangt.

Die Lösung der Aufgabe erfolgt durch die kennzeichnenden Merkmale des Patentanspruchs 1.

Der Nachweisstoff selbst wird als Substrat katalytisch umgesetzt und es ist eine quantitative, gasartspezifische Bestimmung einer Konzentration oder auch einer Dosismenge möglich. Die Enzym-Katalyse läuft jetzt nur bei Anwesenheit des Substrat-Nachweisstoffes ab, so daß während der Meßbereitschaft kein Enzym verbraucht wird. Dadurch reicht ein großer Vorrat für eine längere Meßzeit oder für die Messung sehr hoher Konzentrationen aus.

Die Auswertung erfolgt dadurch, daß die Enzymreaktion zu einem verfärbten Reaktionsprodukt führt. Für eine solche kolorimetrische Messung ist vorgesehen, daß zur Bildung eines verfärbten Reaktionsproduktes der Speicher sowohl ein auf den Nachweisstoff als Enzymsubstrat empfindliches Enzym als auch im Überschuß ein an der Reaktion teilnehmendes Chromogen-Reagenz in Lösung enthält, desssen Eigenschaftsänderung an einer Verfärbung erkennbar ist, deren Intensität von der Menge des nachgewiesenen Enzymsubstrates abhängig ist. Dadurch ist eine selektive kolorimetrische Dosismessung für toxische Gase und Aerosole möglich. Die Dosisbestimmung erfolgt einfach durch Farbvergleich mit bekannten Farbstandards oder durch eine reflektometrische Auswertung in einem Labor. Das Nachweisgerät kommt ohne zusätzliche Energieversorgung aus, ist somit leicht tragbar und kann vom Geräteträger über mehrere Stunden hinweg eingesetzt werden.

Bei der Farbstoffbildung der enzymatischen Reaktion kann sich entweder das Chromogen-Reagenz selbst verfärben, oder das Chromogen-Reagenz bildet mit einer weiteren Farbstoffvorstufe nach erfolgter Reaktion einen Farbstoff. Der Ablauf der enzymatischen Reaktion ist dabei so schnell, daß bei Anwesenheit eines Enzymsubstrats das Enzym lediglich als ein Biokatalysator wirkt, um einen Farbstoff zu erzeugen.

Der poröse Speicher ist in Form einer durchscheinenden Füllung einer Küvette ausgebildet, die die Reagentienlösung mit dem Enzym enthält und über eine für den Nachweisstoff als Substrat durchlässige Öffnung der Küvette mit der Umgebung verbunden ist. Man erhält auf diese Weise ein plakettenförmiges oder röhrchenförmiges kolorimetrisches Dosimeter, das sich bei Exposition zum Nachweisstoff verfärbt.

Die Ausbreitung der Farbreaktion ist auf die Trägeroberfläche begrenzt, da die Kapillarkraft der aus dem Reservoir über die poröse Füllung nachsteigenden Lösung eine Rückdiffusion in die Füllung verhindert. Es findet somit keine Verfärbungsschwächung durch abdiffundierende Farbstoffe statt. Die Konzentration des gebildeten Farbstoffes an der Oberfläche des Enzymträgers begünstigt die visuelle Auswertbarkeit der Farbintensitätsveränderung. Durch die Verhinderung der Rückdiffusion des Farbstoffes können nunmehr auch solche Chromogen-Reagenzien eingesetzt werden, die einen löslichen Farbstoff bilden. Damit ist die mögliche einsetzbare Farbstoffpalette für eine kolorimetrische Dosismessung nicht mehr auf unlösliche Farbstoffe beschränkt. Diese Tatsache erweitert den Einsatzbereich des Dosimeters auf den Nachweis einer Vielzahl von toxischen Gasen und/oder Aerosolen.

Ein einfacher Anbringungsort für das Enzym ist dasjenige Ende der porösen Füllung, welches dem Nachweisstoff zugewandt ist. Das Enzym kann zweckmäßigerweise in lyophilisierter Form oder auch adsorptiv bzw. kovalent an dem Ende der porösen Füllung aufgetragen sein, welches nunmehr als Träger für das Enzym dient. Die Lösung aus dem Reservoir steigt in der porösen Füllung auf und aktiviert das an dessen Ende befindliche Enzym und konzentriert es an der Oberfläche. Eine Rückdiffusion in die poröse Füllung hinein wird durch die Kapillarkraft der aufsteigenden Lösung verhindert. Auch die gebildeten löslichen wie unlöslichen Farbstoffe verbleiben auf der dünnen Oberflächenschicht der porösen Füllung und sind somit einer visuellen Auswertung besonders zugänglich.

Eine weitere zweckmäßige Ausführungsform wird dadurch erhalten, daß das Enzym in der Lösung des Reservoirs aufgenommen ist und die poröse Füllung als Träger des Enzyms vorgesehen ist. Bei einer derartigen Ausgestaltung kann man die Lösung in dem Reservoir getrennt zubereiten und lagern, und erst bei Bedarf die poröse Füllung mit dem Reservoir in Verbindung bringen, wodurch die Nachweisreaktion an der dem Nachweisstoff zugewandten Ende der porösen Füllung eingeleitet wird.

Eine andere günstige Möglichkeit, das Enzym auf einem Träger anzubringen, besteht darin, es z.B. kovalent an eine die poröse Füllung abdeckende Membran zu binden.

Als poröse Füllung kommen poröse Glasstäbe mit planen Flächen an ihren Enden mit einer typischen Größe von 20 mm Länge und einem Durchmesser von 6 mm in Frage. Sie werden in geeignete Glasgefäße als Reservoire gestellt, die einen Innendurchmesser von 8 mm und eine Höhe von 24 mm haben. In diese wird die Lösung mit dem Reagenzien oder auch mit dem Enzym zusammen eingefüllt. Der eingetauchte poröse Glasstab saugt die Lösung auf und bringt sie an die dem Nachweisstoff zugewandten Oberfläche des Glasstabes. Dort kann das Enzym mit einem vorhandenen Enzymsubstrat reagieren und zu einer Verfärbung der in der Lösung vorhandenen Farbstoffvorstufen führen.

Eine besonders günstige Bereitstellung des Nachweisgerätes wird dadurch erreicht, daß das Enzym an der dem Nachweisstoff auszusetzenden Seite und die Reagenzien an der dem Nachweisstoff abgewandten Seite der porösen Füllung in lyophilisierter Form aufgenommen und nach Herstellung der Verbindung zu dem Reservoir, welches der Mobilisierung der lyophilisierten Reaktionspartner dienende Lösungsvermittler enthält, zu der enzymatischen Reaktion befähigt sind. Auf diese Weise hat man die poröse Füllung als Träger für das Enzym einerseits und die für die enzymatische Reaktion erforderlichen Reagenzien andererseits an räumlich verschiedenen Stellen untergebracht, wodurch die enzymatische Reaktion solange inaktiv bleibt, bis die poröse Füllung in das Reservoir eingetaucht wird, welches Lösungsvermittler enthält, die die lyophilisierten Reaktionspartner mobilisieren. Erst danach ist das Nachweisgerät für eine dosimetrische Messung einsatzbereit. Als Lösungsvermittler kommen Detergentien in Frage, die Gruppen von kationenaktiven, anionenaktiven oder nicht ionogenen Tensiden enthalten. Solche Detergentien sind beispielsweise unter dem Handelsnamen Triton (eingetragenes Warenzeichen) bekannt.

Sollen große Mengen von Nachweisstoffen über eine längere Zeit gesammelt werden, wobei ein erhöhter Verbrauch der an der enzymatischen Reaktion beteiligten Reaktionspartner zu einem unerwünscht frühen Erschöpfen des Nachweisgerätes führen würde, ist es zweckmäßig, die poröse Füllung von der den Nachweisstoff enthaltenden Umgebung mit einer gasdurchlässigen Membran abzudecken. Die Membran dient dann als Diffusionsbarriere und bestimmt die Zugänglichkeit des Enzymsubstrats zu dem Enzym. Auf diese Weise läßt sich auch eine Dosimetrie bei höheren Konzentrationen über einen längeren Zeitraum durchführen, die sonst in kurzer Zeit zu einer tiefen, nicht mehr weiter auswertbaren Verfärbung führen würde. Andererseits läßt sich mit einem Farbreagenz, das nur eine geringe Farbtiefe ergibt, eine Langzeitmessung bei höheren Konzentrationen des Nachweisstoffes durchführen. Indem man eine poröse Füllung, die eine solche Deckmembran besitzt, mit einer porösen Füllung ohne Deckmembran kombiniert, lassen sich bei geeigneter Zusammenstellung von Enzym und Farbstoffreagenzien sowohl eine Langzeitmessung als auch eine Kurzzeitmessung in ein- und demselben Nachweisgerät verwirklichen. Dabei tauchen die beiden porösen Füllungen in jeweils ihnen zugeordnete getrennte Reservoire mit unterschiedlichen Lösungen ein.

Zum Nachweis von beispielsweise Wasserstoffperoxid als Substrat für das Enzym Meerrettich-Peroxidase wird eine Lösung aus je 1mMol/l 4-Aminoantipyrin und N-Ethyl-N-Sulfopropyl-m-Toluidin in einem 50 mMol/l Phosphatpuffer als Farbstoffreagenzien angesetzt. Der Phosphatpuffer soll einen pH-Wert von 7.3 gewährleisten. Mit einem derartigen Ansatz ist Wasserstoffperoxid selektiv über die Peroxidase (POD) als Biokatalysator durch eine Farbreaktion nachweisbar. Dabei bilden Aminoantipyrin und Ethyl-Sulfopropyltoluidin die Chromogene, welche aufgrund der Enzymreaktion zu einem Farbstoff kombinieren.

Der Nachweis von Wasserstoffperoxid mit einem Chromogen als Farbstoffreagenz, welches direkt infolge der Enzymreaktion in einen Farbstoff umgewandelt wird, erfolgt durch eine Lösung, die sowohl zwei internationale Einheiten des Enzyms Meerrettich-Peroxidase (POD) als auch als Farbstoffreagenz ein millimolare 2,2′-Azino-bis -(3-Ethylbenzthiazolin-6-Sulfonsäure) in einem 0,1 molaren TRIS-HCl-Puffer pH 8.0 enthält.

Weitere Beispiele für den Nachweis von Gasen über eine enzymatische Reaktion sind folgende:
Zur Langzeitmessung von Wasserstoffperoxid wird ein unempfindlicheres Farbsystem verwendet, wie es z. B. aus einer Lösung von 1 mMol/l 4-Chlor-1-Naphtol in 50 mMol/l TRIS-HCl und 0,2 Mol/l Natrium-Chloridlösung besteht.

Acetaldehyd wird mit Aldehyddehydrogenase (AlDH) in der Weise gemessen, daß zunächst das Enzym von Blindreaktionen hervorrufenden Stabilisatoren befreit wird, indem man es mit Ammoniumsulfat fällt und in 0,1 molaren Kaliumphosphatpuffer pH 8.0 aufnimmt. Ein poröser Glasstab wird an seinem einen Ende mit 0,05 mL einer Lösung von Diaphorase (4 Millieinheiten pro mL) und ALDH (3 Millieinheiten pro mL) in gleichem Puffer betropft und mit dem entgegengesetzten Ende in ein Glasgefäß als Reservoir gestellt. Das Reservoir enthält 0,3 mL einer Lösung mit 0,1 N-Kaliumphosphat pH 9.0, 1,6 mMol/l Nikotinamid-Adenin-Dinukleotid (NAD) und 0,3 Millimol Jod-Nitrotetrazoliumchlorid (INT). Setzt man den porösen Glasstab einer Atmosphäre mit Acetaldehyd aus, können nach einer Expositionszeit von 5 Minuten Konzentrationen von kleiner 5 bis 100 ppm Acetaldehyd nachgewiesen werden.

Zur Bestimmung von Formaldehyd mit Formaldehyddehydrogenase wird das Enzym in 50 mMol/l Phosphatpuffer pH 7.4 (10 U/ml) gelöst. Poröse Glasstäbe werden kurz in die Enzymlösung getaucht und an der Luft 10 Minuten getrocknet. Das dem Nachweisstoff zugewandte Ende wird mit einem Silikonschlauch von etwa 10 Millimeter Länge umgeben. Danach wird der Stab mit seinem anderen Ende in ein etwa 24 mm hohes Glasgefäß gestellt, welches eine Lösung folgender Zusammensetzung enthält: 50 mMol/l Phosphatpuffer pH 7.4; 0,2 mMol/l INT; 0,05 mMol/l Methoxyphenazin Methosulfat; 1 mMol/l NAD. Auf diese Weise läßt sich Formaldehyd in der Gasphase nach einer Expositionszeit von etwa 10 Minuten bei Konzentrationen von kleiner 1 ppm erfassen.

Ein Ausführungsbeispiel der Erfindung wird anhand einer schematischen Darstellung gezeigt und im folgenden näher erläutert.

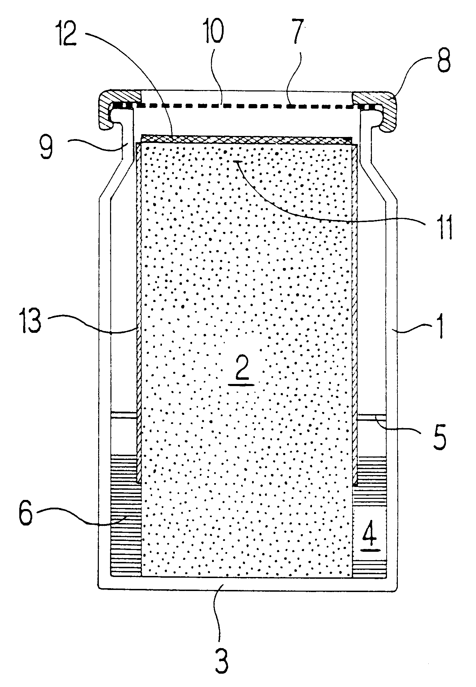

In der einzigen Figur ist im Schnitt ein einseitig geöffnetes Glasgefäß (1) dargestellt, welches einen zylindrischen porösen Körper (2) aufnimmt, der von der Öffnung des Glasgefäßes (1) bis zu dessen Boden (3) reicht. Der poröse Körper (2) besteht ebenfalls aus Glas und taucht mit seinem dem Gefäßboden (3) zugewandten Ende in eine Lösung (4) ein, welche die für die enzymatische Reaktion notwendigen Reagenzien wie Farbstoffvorstufen und Kofaktoren in einer Pufferlösung enthält. Die Lösung (4) ist in einem durch einen ringförmigen Absatz (5) abgeteilten Reservoir (6) enthalten. Der Absatz (5) ist entlang der Innenwand des Gefäßes (1) befestigt und hindert zum einen die Lösung (4) daran, aus dem Gefäß (1) zu entweichen, und dient andererseits dazu, den porösen Körper (2) in seiner Position zu halten. Die Öffnung des Glasgefäßes (1) ist mit einer porösen Membran (7) als Diffusionsbarriere verschlossen. Zur Fixierung der Membran (7) ist ein gummielastischer Kragen (8) um eine Wulst (9) der Gefäßöffnung gespannt. Das den Porenöffnungen (10) der Membran (7) und somit der den Nachweisstoff enthaltenden Umgebung zugewandte Ende (11) des porösen Glasstabes (2) ist mit einer Membran (12) abgeschlossen, welches als Träger für das kovalent gebundene Enzym dient. Zur Verbesserung der Kapillardiffusion der Lösung (4) aus dem Reservoir (6) zu dem Träger (12) ist der Glaskörper (2) zumindest an seiner aus dem Reservoir (6) austretenden Oberfläche mit einem Silikonschlauch (13) überzogen. Der Silikonschlauch (13) ist weiterhin über dem Absatz (5) hinaus in das Reservoir (6) weitergeführt, wodurch er gleichzeitig eine Dichtfunktion für das Reservoir (6) ausübt.

## Patentansprüche

1. Nachweisgerät für gasförmige Stoffe und Aerosole mit Hilfe einer enzymatischen Reaktion, die zwischen einem Substrat und einem Enzym abläuft, welches einerseits dem nachzuweisenden Stoff ausgesetzt und andererseits von einem Träger aufgenommen ist, der als ein poröser Speicher (2, 6, 11, 12) mit einer die enzymatische Reaktion aktivierenden und erhaltenden Lösung (4) in Verbindung ist, deren Eigenschaftsänderungen durch die enzymatische Reaktion auswertbar sind, wobei der Nachweisstoff als ein Substrat durch die enzymatische Reaktion stöchiometrisch in ein der quantitativen Bestimmung des Nachweisstoffes dienendes Reaktionsprodukt überführbar ist, und der Speicher (2,6,11,12) zur Bildung eines verfärbten Reaktionsproduktes sowohl ein auf den Nachweisstoff als Enzymsubstrat empfindliches Enzym als auch ein an der Reaktion teilnehmendes Chromogen-Reagenz enthält, dessen Eigenschaftsänderung an einer Verfärbung erkennbar ist, deren Intensität von der Menge des nachgewiesenen Enzymsubstrates abhängig ist, dadurch gekennzeichnet, daß der Speicher eine durchscheinende, poröse Füllung (2) in einer Küvette (1) besitzt, welche in ein zumindest die für die Farbreaktion notwendige Reagenzlösung (4) im Überschuß enthaltendes Reservoir (6) in der Küvette (1) eintaucht, und daß die Küvette (1) mit einer dem Nachweisstoff ausgesetzten porösen Öffnung (7) versehen ist.

2. Nachweisgerät nach Anspruch 1, dadurch gekennzeichnet, daß das Enzym auf dem dem Nachweisstoff zugewandten Ende (11) der porösen Füllung (2) aufgenommen ist.

3. Nachweisgerät nach Anspruch 1, dadurch gekennzeichnet, daß das Enzym als Bestandteil der Reagenzienlösung (4) vorgesehen ist.

4. Nachweisgerät nach Anspruch 2, dadurch gekennzeichnet, daß das Enzym an einer die poröse Füllung (2) abdeckenden und dem Nachweisstoff zugewandten Membran (12) als Träger gebunden ist.

5. Nachweisgerät nach einem der Ansprüche 2 oder 4, dadurch gekennzeichnet, daß das Enzym an dem dem Nachweisstoff zugewandten Ende (11) und die Reagenzien an der dem Nachweisstoff abgewandten Seite der porösen Füllung (2) in lyophilisierter Form aufgenommen sind, und daß nach Herstellung der Verbindung zwischen der porösen Füllung (2) und dem Reservoir (6) welches die der Mobilisierung der lyophilisierten Reaktionspartner dienenden Lösungsvermittler enthält, das Enzym mit den Reagentien zu der enzymatischen Reaktion befähigt ist.

6. Nachweisgerät nach einem der Ansprüche 1 bis 5, dadurch gekennzeichnet, daß die poröse Füllung (2) von der den Nachweisstoff enthaltenden Umgebung mit einer porösen Diffusionsmembran (7) abgedeckt ist.

7. Nachweisgerät nach einem der Ansprüche 2 bis 6, dadurch gekennzeichnet, daß die poröse Füllung (2) an seinem dem Nachweisstoff zugewandten Ende (11) mit einem Silikonschlauch (13) überzogen ist.

8. Nachweisgerät nach einem der Ansprüche 1 bis 7, dadurch gekennzeichnet, daß das zum Nachweis von Wasserstoffperoxid als Substrat für das Enzym Meerrettich-Peroxidase die Lösung je ein mMol/l 4-Aminoantipyrin und N-Ethyl-N-Sulfopropyl-m-Toluidin in 50 mMol/l Phosphatpuffer pH 7.3 als Farbstoffreagenzien und 2 U pro ml Peroxidase enthält.

9. Nachweisgerät nach einem der Ansprüche 1 bis 7, dadurch gekennzeichnet, daß zum Nachweis von Wasserstoffperoxid die Lösung sowohl 2 U pro ml (Internationale Einheiten) des Enzyms Meerrettich-Peroxidase als auch als Farbstoffreagenz ein mMol/l 2,2'-Azino- bis -(3-Ethylbenzthiacolin-6-Sulfonsäure) in 0,1 Mol/l TRIS-HCl-Puffer pH 8.0 enthält.

## Claims

1. Detection apparatus for gaseous substances and aerosols using an enzymatic reaction which occurs between a substrate and an enzyme which, on the one hand, is exposed to the substance to be detected and, on the other hand, is accommodated by a carrier which as a porous store (2, 6, 11, 12) communicates with a solution (4) which activates and maintains the enzymatic reaction and the changes in properties of which as a result of the enzymatic reaction can be evaluated, with it being possible to convert the detection substance as a substrate by means of the enzymatic reaction stoichiometrically into a reaction product which is used for the quantitative determination of the detection substance, and with the store (2, 6, 11, 12) for the purpose of forming a colour-changed reaction product containing both an enzyme which is sensitive to the detection substance as an enzyme substrate and a chromogen reagent which takes part in the reaction and the change in properties of which can be recognized by a change in colour the intensity of which is dependent upon the quantity of the enzyme substrate detected, characterised in that the store has a transparent, porous filling (2) in a cuvette (1), which filling dips into a reservoir (6) in the cuvette (1) containing to excess at least the reagent solution (4) necessary for the colour reaction, and in that the cuvette (1) is provided with a porous opening (7) exposed to the detection substance.

2. Detection apparatus according to claim 1, characterised in that the enzyme is accommodated at the end (11) of the porous filling (2) that faces the detection substance.

3. Detection apparatus according to claim 1, characterised in that the enzyme is provided as a constituent of the reagent solution (4).

4. Detection apparatus according to claim 2, characterised in that the enzyme is bound to a membrane (12) as a carrier that covers the porous filing (2) and faces the detection substance.

5. Detection apparatus according to one of the claims 2 or 4, characterised in that the enzyme is accommodated at the end (11) which faces the detection substance and the reagents are accommodated in a lyophilized form on the side of the porous filling (2) that is remote from the detection substance, and in that after the connection has been established between the porous filling (2) and the reservoir (6) which contains the dissolving intermediaries used for the mobilization of the lyophilized coreactants, the enzyme with the reagents is made capable of the enzymatic reaction.

6. Detection apparatus according to one of the claims 1 to 5, characterised in that the porous filling (2) is shielded from the environment containing the detection substance with a porous diffusion membrane (7).

7. Detection apparatus according to one of the claims 2 to 6, characterised in that the porous filling (2) is covered with a silicon tube (13) at its end (11) facing the detection substance.

8. Detection apparatus according to one of the claims 1 to 7, characterised in that for the detection of hydrogen peroxide as a substrate for the enzyme horseradish peroxidase the solution contains one mMol/l of each of 4-aminoantipyrine and N-ethyl-N-sulphopropyl-m-toluidine as dye reagents in 50 mMol/l phosphate buffer pH 7.3 and 2 U per ml peroxidase.

9. Detection apparatus according to one of the claims 1 to 7, characterised in that for the detection of hydrogen peroxide the solution contains not only 2 U per ml (International Units) of the enzyme horseradish peroxidase, but also as a dye reagent one mMol/l 2,2'-azino- bis -(3-ethylbenzothiacoline-6-sulphonic acid) in 0.1 mol/l TRIS-HCl-buffer pH 8.0.

## Revendications

1. Dispositif de détection de substances gazeuses et d'aérosols à l'aide d'une réaction enzymatique se produisant entre un substrat et une enzyme, exposé d'une part à la substance à détecter et logé, d'autre part, dans un support qui est en liaison, en tant que réservoir poreux (2, 6, 11, 12) avec la solution (4) activant et entretenant la réaction enzymatique, dont la modification des propriétés peut être évaluée par la réaction enzymatique, la substance à détecter, en tant que substrat, pouvant être transformée de façon stoechiométrique, par la réaction enzymatique, en un produit réactionnel servant à la détermination quantitative de la substance à détecter, et le réservoir (2, 6, 11, 12), pour la formation d'un produit réactionnel coloré, contenant une enzyme sensible à la substance à détecter en tant que substrat enzymatique et un réactif chromogène participant à la réaction, dont la modification des propriétés peut être décelée du fait d'une coloration, dont l'intensité dépend de la quantité de substrat enzymatique détectée, caractérisé en ce que le réservoir contient une charge (2) poreuse transparente dans une cuvette (1), qui est immergée dans un réservoir (6), présent dans le récipient en verre (1) contenant, en excès, au moins la solution réactionnelle (4) nécessaire pour la réaction de coloration, et en ce que le récipient en verre (1) est pourvu d'une ouverture (7) poreuse exposée à la substance à détecter.

2. Dispositif de détection selon la revendication 1, caractérisé en ce que l'enzyme est appliquée sur l'extrémité (11) de la charge poreuse (2) orientée en direction de la susbtance détectrice.

3. Dispositif de détection selon la revendication 1, caractérisé en ce que l'enzyme est prévue comme composant de la solution réactionnelle (4).

4. Dispositif de détection selon la revendication 2, caractérisé en ce que l'enzyme est liée, en tant que support, à une membrane recouvrant la charge poreuse (2) et orientée en direction de la substance à détecter.

5. Dispositif de détection selon l'une des revendications 2 ou 4, caractérisé en ce que l'enzyme est déposée à l'extrémité (11) orientée en direction de la substance à détecter et les réactifs sont appliqués sous forme lyophilisée sur la face de la charge poreuse (2) orientée à l'opposé de la substance à détecter et en ce que, une fois établie la communication entre la charge poreuse (2) et le réservoir (6), qui contient les agents de solubilisation servant à la mobilisation des partenaires réactionnels lyophilisés, l'enzyme, avec les réactifs, est rendue apte à la réaction enzymatique.

6. Dispositif de détection selon l'une des revendications 1 à 5, caractérisé en ce que la charge poreuse (2) est isolée de l'atmosphère contenant la substance à détecter au moyen d'une membrane de diffusion (7) poreuse.

7. Dispositif de détection selon l'une des revendications 2 à 6, caractérisé en ce que la charge poreuse (2) est recouverte, au niveau de son extrémité (11) orientée en direction de la substance à détecter, par un tuyau en silicone (13).

8. Dispositif de détection selon l'une des revendications 1 à 7, caractérisé en ce que, pour la détection du peroxyde d'hydrogène en tant que substrat pour l'enzyme de peroxydase de radis noir, la solution contient respectivement 1 mMol/l d'amino-4-antipyrine et de N-éthyl-N-sulfopropyle-m-toluidine dans 50 mMol/l d'un tampon phosphate à pH 7,3 comme réactif de coloration et 2 unités par ml de peroxydase.

9. Dispositif de détection selon l'une des revendications 1 à 7, caractérisé en ce que, pour la détection du peroxyde d'hydrogène, la solution contient 2 U par ml (unités internationales) de l'enzyme peroxydase de radis noir et, en tant que réactif de coloration, 1 mMol/l d'acide 2,2'-azino-bis-(3-éthylbenzthiacolin-6-sulfonique) dans 0,1 Mol/l d'un tampon TRIS-HCl à pH de 8,0.
